# EUROPEAN PATENT SPECIFICATION

(11) **EP 2 300 062 B1**
(45) Date of publication and mention of the grant of the patent: **04.09.2019**
(21) Application number: 09773814.0
(22) Date of filing: 29.06.2009
(51) Int. Cl.: A61L 15/32, A61L 26/00, C08F 251/00, C08F 289/00, C08L 51/02, C08L 51/08

(54) **USE OF CROSSLINKED CHITOSAN POLYMER FOR HEPARIN REMOVAL**
BENUTZUNG VON VERNETZTEN CHITOSANPOLYMER FÜR DAS WEGSCHAFFEN VON HEPARIN.
UTILISATION D'UN POLYMÈRE DE CHITOSAN RÉTICULÉ POUR LA SÉPARATION DE L'HÉPARINE

(30) Priority: 02.07.2008 PL 38557308
(43) Date of publication of application: 30.03.2011
(73) Proprietor: Uniwersytet Jagiellonski, 31007 Krakow (PL)
(72) Inventor: KAMINSKI, Kamil, 30-733 Kraków (PL); ZAZAKOWNY, Karolina, 32-040 Ochojno (PL); SZCZUBIALKA, Krzysztof, PL-32-442 Krzywaczka (PL); NOWAKOWSKA, Maria, PL-30-433 Krakow (PL)
(74) Representative: Padée, Grazyna
(86) International application number: PCT/PL2009/000070
(87) International publication number: WO 2010/002281

(56) References cited:
- EP-A- 1 260 237
- WO-A-96/02258
- WO-A-98/19718
- WO-A-2007/100588
- US-A- 4 326 532
- US-A- 5 116 824
- US-B1- 6 608 040

## Description

The subject of the invention is the use of crosslinked chitosan for removal of heparin from blood and other physiological fluids.

Heparin, a substance discovered by McLean almost a century ago, found clinical applications since 1937 and is a first polysaccharide-based drug, which is widely applied in the therapy of humans. Heparin is a complex mixture of glucosaminoglycans (GAGs) of high degree of sulfation (with 2.7 negative charges in a disaccharide repeating unit it has the highest density of the negative charge among biological molecules) which is produced in the mast cells of animals (e.g. in bovine intestines or porcine lungs). It shows very strong anticoagulant action, although only one third of heparin molecules shows anticoagulant properties. Its action is based on enhancing the ability of antithrombin (AT) to deactivate thrombin and Xa factor, enzymes responsible for blood clotting. Therefore, heparin is a drug of choice in situations, when a fast anticoagulant effect is necessary, e.g. during surgical procedures, particularly to prevent blood coagulation in the apparatus used in extracorporeal therapy such as dialysers and oxygenators. It also has many other therapeutical applications, e.g. in the treatment of unstable angina pectoris or acute myocardial infarction.

However, administration of heparin involves many adverse effects, among which the most frequent are bleeding, heparin induced thrombocytopenia (HIT), and osteoporosis.

Therefore, it is often necessary to remove heparin from the bloodstream after its desired anticoagulant effect is obtained. A series of method of its removal has been developed. Usually this is achieved by the administration of protamine, a protein introduced to the clinical practice as a heparin antagonist almost simultaneously with heparin (Fischer, A Biochem Zeit. 278, 133, 1935). It shows high content of basic aminoacids (such as arginine, lysine, and histidine) which may reach 80%. Another polymer used to remove heparin is poly-L-lysine (Ma, X., Mohammad, S.F., Kim, S.W. Biotechnology and Bioengineering Volume 40, Issue 4, 5 August 1992, Pages 530-536), which is used also to enhance protamine action. Yet another approach to the problem of heparin removal is its enzymatic degradation using immobilized heparinise (Kolde, H.-J., Pelzer, H., Borzhenskaya, L., Russo, A., Rose, M., Tejidor, L. Hamostaseologie Volume 14, Issue 1, 1994, Pages 37-43).

Unfortunately, the above mentioned methods of heparin removal may themselves induce side effects. Protamine, if left in the bloodstream, may result in adverse reactions in about 10% of patients. They may be very severe, even fatal, and include pulmonary hypertension, arterial hypotension, anaphylactic shock, thrombocytopenia, granulocytopenia, complement activation and cytokine release. Removal of heparin with protamine is not complete and is accompanied with allergic reactions. On the other hand, poly-L-lysine is still quite an expensive polymer.

Many attempts to construct devices for heparin removal have been undertaken, mostly based on the application of immobilized poly-L-lysine (Joseph B. Zwischenberger, MD, Roger A. Vertrees, BA, CCP, Robert L. Brunston, Jr., MD, Weike Tao, MD, Scott K. Alpard, MD, and Paul S. Brown, Jr., MD, The Journal of Thoracic and Cardiovascular Surgery 1998 Volume 115, Number 3; Zwischenberger, J.B., Tao, W., Deyo, D.J., Vertrees, R.A., Alpard, S.K., Shulman, G.Annals of Thoracic Burgery Volume 71, Issue 1, 2001, Pages 270-277). The heparin removal device (HRD) described in the above papers was extracorporeally included in the patient's bloodstream by veno-venous circuit. It separates plasma, which upon heparin removal by contact with poly-L-lysine, is returned to the patient's blood. Despite the promising experimental data, there has been only limited experience with such heparin removal devices, and none of them have been clinically implemented until now. A method frequently used to avoid complications brought about by free heparin antagonists is their immobilization on polymeric supports inside the heparin removal devices. For example, protamine was supported on a matrix obtained by grafting an acrylic polymer onto cellulose (Hou, K.C., Roy, S., Zaniewski, R., Shumway, E. Artificial Organs Volume 14, Issue 6, 1990, Pages 436-442) or inside cellulose fibres (Wang, T., Byun, Y., Kim, J.-S., Liang, J., Yang, V.C. International Journal of Bio-Chromatography Volume 6, Issue 2, 2001, Pages 133-149). It was shown that the bioreactor removed more than 50% of the administered blood during 10 minutes at the blood flow rate of 100 ml/min. While fast injection of protamine in dogs results in acute hypotension, application of a bioreactor containing immobilized protamine did not result in any statistically significant changes in monitored hemodynamic parameters. Another paper reports efficient removal of heparin using beads obtained from alginate and poly-L-lysine (M. Sunil Varghese, D. Hildebrandt, and D. Glasser, N. J. Crowther, D. M. Rubin, Artificial Cells, Blood Substitutes, and Biotechnology, 34: 419-432, 2006).

The interaction between chitosan (Formula 4, where R denotes H or COCH₃) and heparin. For example, nanospheres were obtained by dropping heparin solution into chitosan solution (] Liu, Z., Jiao, Y., Liu, F., Zhang, Z. (2007) Journal of Biomedical Materials Research - Part A, 83 (3), pp. 806-812) and microspheres of chitosan crosslinked with epichlorohydrin covered with heparin by immersing in its solution. However, there are no reports on the application of chitosan polymers for removal of heparin from blood or other physiological fluids.

International patent application WO9602258 discloses the use of chitosan with heparin attached to it to produce an anti-adhesive agent, intended to prevent or to significantly reduce unwanted adhesion of tissue surrounding the damaged tissue. Immobilization of heparin in chitosan requires reaction conditions of a pH below 7.

A chitosan polymer cross-linked with genipin is known from patent application EP1260237. The aim of such cross-linking is improvement of mechanical properties, thermal stability and biocompatibility of the polymer, as well as reduction or elimination of its toxicity. The cross-linked chitosan polymer can be used to produce implants or wound dressings.

In patent application WO2007100588 a chitosan polymer cross-linked with genipin is disclosed, which forms a hydrogel layer on an amperometric biosensor. Biosensor is suitable for determination of blood components.

The subject of the invention is the use of the chitosan (Ch) polymer crosslinked with genipin (Gp) with a general structure given in Formula 1, 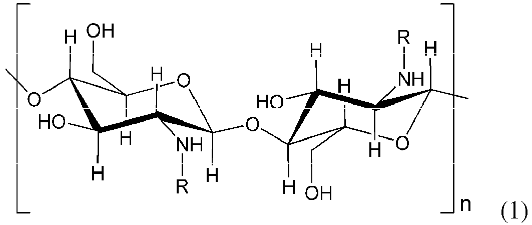 wherein R denotes X group with the formula 2 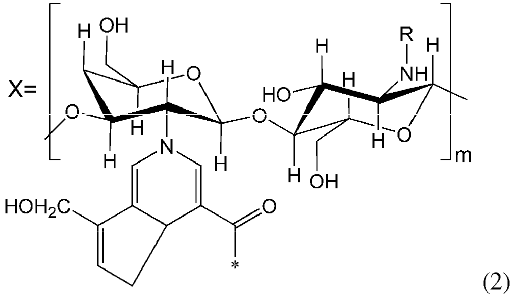 or with the formula 3, 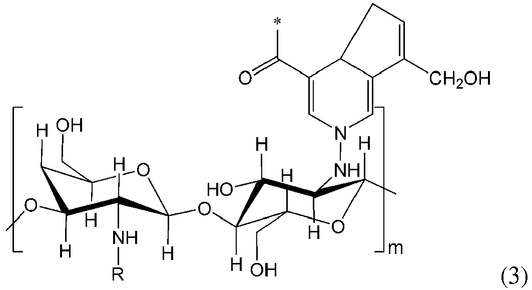 or R denotes -CH₂CH(OH)CH₂N⁺(CH₃)₃, provided that a part of substituents R denotes X and other part of substituents R denotes -CH₂CH(OH)CH₂N⁺(CH₃)₃, and n and m are natural numbers, as a stationary phase in the heparin removal devices for the removal of heparin from blood and physiological liquids of mammals.

The polymer is preferably applied in the form of microspheres. These are hydrogel microspheres obtained in the reaction in reverse emulsion. Hydrogel microspheres of chitosan polymer crosslinked with genipin are obtained by mixing chitosan solution and an organic solvent immiscible with water, preferably cyclohexane, and then the microspheres are crosslinked by adding the solution of genipin and heating at the temperature at which water and the organic solvent are liquid, preferably at 60°C. In order to obtain a polymer, where R is CH₂CH(OH)CH₂N⁺(CH₃)₃ group, the microspheres described above are reacted with glycidyltrimethylammonium chloride (Gl).

Polymer according to the invention is preferably used also in the form of a film. The polymer in the form of a film is obtained by adding genipin to the solution of chitosan, which is poured onto a surface and kept at 0-100°C, preferably at 50°C till the sufficient degree of crosslinking is achieved. In order to obtain a film of a polymer, where R denotes - CH₂CH(OH)CH₂N⁺(CH₃)₃ the film described above is reacted with glycidyltrimethylammonium chloride.

According to the invention, the polymer finds application as a stationary phase in the heparin removal devices. The chitosan polymers crosslinked with genipin (ChGp) bind heparin in the aqueous solution. The rate and efficiency of binding depend on pH and decrease with increasing pH of the solution. However, at pH 7.4, characteristic of blood, heparin binding may be in some cases to slow and inefficient. Genipin-crosslinked chitosan microspheres substituted with glycidyltrimethyl ammonium chloride (ChGpGl microspheres) show significantly increased efficiency and rate of heparin adsorption at pH 7.4. Glycidyltrimethyl ammonium chloride (GTMAC) does not undergo pH-dependent protonation, therefore it provides the macromolecule with stable positive charge, even at high pH values. Moreover, chitosan substituted with GTMAC displays even stronger antibacterial and antifungal activity compared to unsubstituted chitosan which is another advantage of the preferable form of the invention.

The rate of the process of heparin adsorption may be adjusted as needed by the application of the polymer in the form of the microspheres and their proper amount. Thus, both types of microspheres may be used to efficiently remove heparin from solution at different pH values.

One of the advantages of the polymer according to the invention is that chitosan and genipin are inexpensive and nontoxic. Chitosan is a well-known biodegradable and biocompatible polymer. It has many biomedical applications in the form of a solution, films, and microspheres. Genipin (Formula 5) is a natural nontoxic crosslinking agent extracted from *Gardenia jasminoides* fruits. Crosslinking with genipin can be easily controlled since the crosslinked polymer is intensively blue. Also surfactants used to stabilize emulsion during microsphere synthesis are known as nontoxic.

The possibility of the desorption of heparin bound to the polymer according to the invention was also studied. It was found that the addition of concentrated solution of NaCl to the suspension of the ChGpGl microspheres containing adsorbed heparin lead to the heparin desorption, therefore the polymer according to the invention may be reused in heparin removal.

The subject of the invention was described in more detail in the examples. In the experiments described in the examples the following materials were used: low molecular chitosan (Ch) (Aldrich), genipin (Gp, Challenge Bioproducts Co., Ltd., 98%), glicydilotrimethylammonium chloride (GTMAC, Fluka, 90%), heparin sodium salt from bovine intestinal mucosa (Sigma), Span 40 (Fluka), Span 80 (Fluka), Azure A (standard, Fluka), potassium chloride (analytical grade, POCh), potassium dihydrogen phosphate (analytical grade, POCh), disodium hydrogen phosphate (analytical grade, POCh), sodium chloride (analytical grade, POCh), cyclohexane (Lach-Ner, analytical grade), and acetic acid (POCh, reagent grade) were used as received. Water was distilled twice and purified using the Millipore SIMPLICITY system.

The results are shown in figures:
Fig. 1 shows the dependence of relative heparin concentration (c₀=200 µg/ml, V=5 ml) on time after 40 mg of ChGp microspheres were added to the buffer solution at pH of 6.0 (●), 6.8 (■), 7.4 (◆), and 8.0 (▲).
Fig. 2 shows the dependence of relative heparin concentration (c₀=200 µg/mL, v = 5 mL) on time for 40 (●) and 150 mg (■) of ChGp microspheres added at pH 6.8 (a) and for 20 (●), 40 (■), and 80 mg (◆) ChGp microspheres added at pH 7.4 (b).
Fig. shows the dependence of relative heparin concentration (c₀=200 µg/ml, V=5 ml) on time after addition of 40 mg of ChGp microspheres (●) and ChGpGl microspheres (■) at pH 7.4.
Fig. 4 shows the dependence of relative heparin concentration (c₀=200 µg/ml, V=5 ml, PBS buffer) on time for 20 (●), 40 (■) and 80 mg (◆) ChGpGl microspheres at pH 7.4.

### Ref. Example 1

### Synthesis of chitosan microspheres crosslinked with genipin (ChGp)

Chitosan (0.5 g) was dissolved in 35 ml of 0.7% v/v acetic acid and then dialyzed against water in order to remove acetic acid. After dialysis the pH value was 5.2. The above chitosan solution was mixed with 250 ml of cyclohexane in a 500 ml round-bottom flask containing 0.5 g of Span 80 surfactant and 0.25 g of Span 40 surfactant. The mixture was emulsified with a mechanical stirrer at 1200 rpm. Then, 1 ml of 5% w/v solution of genipin in 70% v/v solution of ethanol was added. The mixture was heated to 60°C and mixing was continued. After 3 hours the water phase became blue, which indicated that the reaction of crosslinking with genipin has occurred. The mixture was left overnight without mixing at room temperature. The chitosan microspheres obtained were washed with an excess of cyclohexane and filtered using a Büchner funnel. The microspheres were dried using a filter paper and stored in a refrigerator tightly closed in order to avoid drying. A part of microspheres was dried at 40°C in a vacuum drier.

### Ref. Example 2

### Synthesis of a chitosan film crosslinked with genipin (ChGp)

Chitosan (0.5 g) was dissolved in 35 ml of 0.7% v/v acetic acid and then dialyzed against water in order to remove acetic acid. After dialysis the pH value was 5.2. 3.5 ml of this solution was mixed with 40 µl of 5% w/v solution of genipin solution in the 70% v/v aqueous solution of ethanol. The mixture received was poured into a Petri dish, covered and heated at 50°C for 2 hours. The film was ready after one day.

### Example 3

### Synthesis of cationically modified chitosan microspheres (ChGpGl)

About 0.25 g of ChGp microspheres were placed for 5 hours in 50 ml of dilute acetic acid at pH about 5. Then the microsphere suspension in the above solution was placed in a 100 ml round-bottom flask and 10 ml of GTMAC was added. The suspension was mixed with a mechanical stirrer and the temperature was kept at 55°C. Microspheres were filtered out under reduced pressure and washed several times with an excess of methanol, and then with a pH 7.4 buffer, which was used in further studies on microspheres.

### Ref. Example 4

### The studies of heparin adsorption by ChGp microspheres

Heparin concentration in the solution was determined spectrophotometrically using Azure A dye. In short, to 0.1 ml of heparin solution added was 0.9 ml of a suitable buffer and 1 ml of 4.0·10⁻⁵ M Azure A solution. The solution was then mixed and its absorption spectrum was measured. Heparin concentration was determined based on the intensity of 630 nm absorption band, which corresponds to monomeric molecules of Azure A.

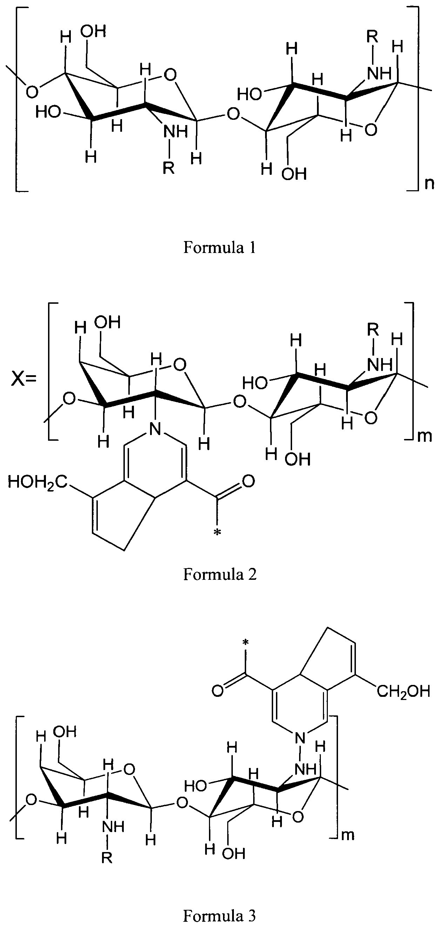

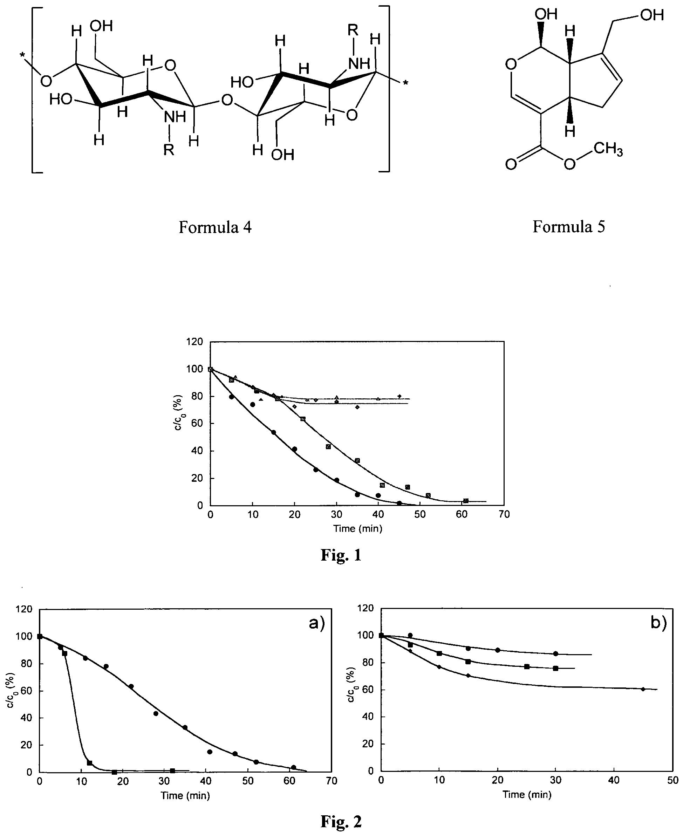

Heparin adsorption by microspheres was studied by adding microspheres to buffered aqueous solutions of heparin and measuring changes of heparin concentration, using a colorimetric method with Azure A as a titrant. It was found that in 5 ml of the pH 6.0 buffer solution the concentration of heparin decreases from the initial value of 200 µg/ml almost to zero during 50 minutes after addition of 40 mg of ChGp microspheres (Fig.1). The amount of heparin adsorbed by ChGp microspheres changes drastically with the change of pH in the range 6.0 - 8.0. Whereas at pH 6.8 heparin may be practically completely removed from 5 ml of the 200 µg/mL solution with 40 mg of ChGp microspheres, at pH 7.4 - 8.0 only about 20% of heparin may become adsorbed at the same conditions.

It was also studied how the amount of ChGp microspheres influences the adsorption profile of heparin and if the amount of heparin adsorbed at higher pH concentrations may be increased by increasing the amount of ChGp microspheres (Fig.2). It was found, that at pH 6.8 the amount of ChGp microspheres added strongly influences the rate of heparin adsorption. The increase in microspheres weight from 40 to 150 mg reduces the time necessary to decrease heparin concentration by 50%. On the other hand, at pH 7.4 adsorption of heparin is much slower, less effective and less sensitive to the amount of ChGp microspheres added. The addition of 80 mg of ChGp microspheres resulted in slight and slow decrease of heparin concentration - down to 60% of its initial value during 50 minutes.

### Example 5

### The studies of heparin adsorption by ChGpGl microspheres

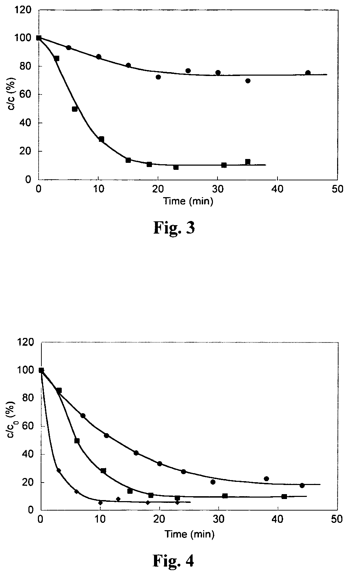

The study was performed using the method described in Example 4. The ability of ChGpGl microspheres to adsorb heparin, both with respect to the adsorption rate and the amount of heparin adsorbed based on the unit weight of microspheres, is significantly improved by substitution of ChGp microspheres with ammonium groups. Fig.3 shows the comparison of heparin adsorption by identical weights of ChGp and ChGpGl microspheres. While at pH 7.4 40 mg of ChGp microspheres may adsorb only about 20% of heparin in 5 ml of the solution containing 200 µg/ml of heparin, ChGpGl microspheres may bind 90% of heparin at the same experimental conditions. The process is very fast; the drop of heparin concentration by 50% occurs during about 5 minutes. The comparable rate of heparin adsorption by ChGp microspheres may be achieved only at significantly lower pH and using several time greater mass of microspheres.

In order to check if the rate and the degree of heparin adsorption may be controlled by the change of the amounts of microspheres used the study was also performed on heparin binding by different amounts of ChGpGl microspheres (Fig. 4). The results show that the rate of heparin adsorption may be increased by the application of a greater amount of ChGpGl microspheres.

### Example 6

### Regeneration of ChGpGl microspheres

80 mg of ChGpGl microspheres were suspended in 5 ml of pH 7.4 buffer containing 1 mg of heparin. Using a spectrophotometric method it was shown that the heparin concentration decreased almost to zero during 10 minutes. The suspension was then centrifuged at 12000 rpm for 3 minutes. 2.5 ml of the solution was removed and replaced with the same volume of 2 M NaCl solution. The suspension was mixed and placed in a sonicator for 5 minutes and centrifuged for next 5 minutes. The solution from above the microspheres was sampled and the heparin concentration was assayed with the spectrophotometric method using Azure A as a dye.

It was shown that in 1 M NaCl solution about 55% of the adsorbed heparin undergoes desorption from ChGpGl microspheres. ChGpGl microspheres may thus be potentially reused to remove heparin.

## Claims

1. Use of a chitosan polymer crosslinked with genipin of a general formula 1 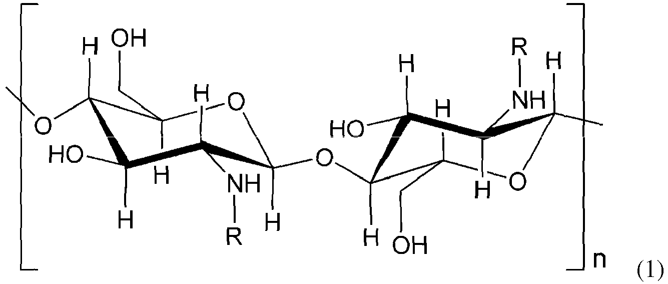 wherein a part of substitutents R denotes -CH₂CH(OH)CH₂N⁺(CH₃)₃ and the other part of of substitutents R denotes X group with the formula 2 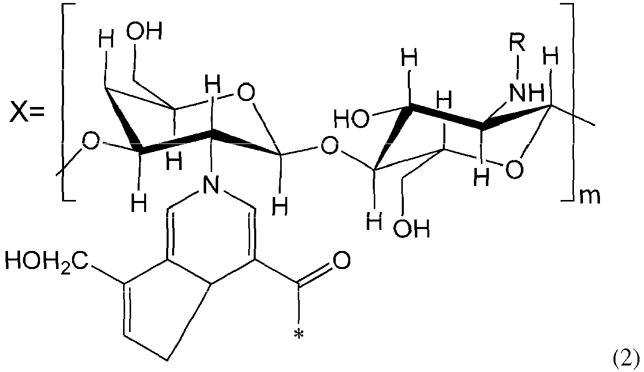 or with the formula 3, 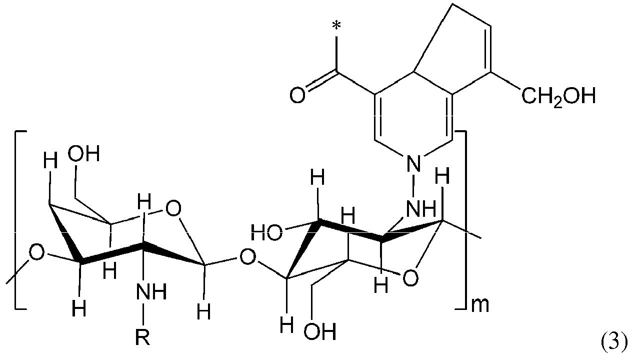 and n and m are natural numbers, as a stationary phase in the heparin removal devices for the removal of heparin from blood and physiological liquids of mammals.

2. Use according to Claim 1, wherein the chitosan polymer, where R denotes -CH₂CH(OH)CH₂N⁺(CH₃)₃ group, is obtained in the reaction of genipin-crosslinked chitosan with glycidyltrimethylammonium chloride.

3. Use according to Claim 1, wherein the crosslinked chitosan polymer is used in the form of microspheres.

4. Use according to Claim 3, wherein the hydrogel microspheres obtained in the reaction in reverse emulsion are used.

5. Use according to Claim 1, wherein the chitosan polymer is used in the form of a film.

## Patentansprüche

1. Verwendung eines Chitosan-Polymers, der mit Genipin aus der allgemeinen Formel 1 quervernetzt ist, 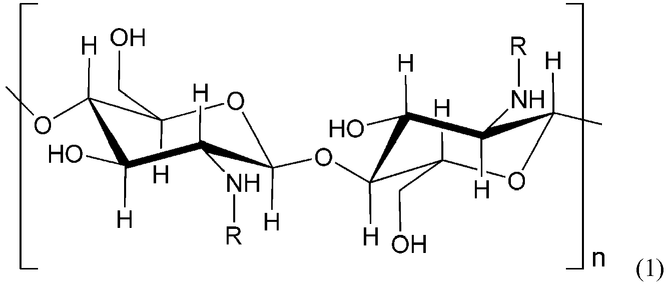 wobei ein Teil der Substituenten R ist -CH₂CH(OH)CH₂N⁺(CH₃)₃ und der andere Teil der Substituenten R beschreibt die Gruppe X aus Formel 2 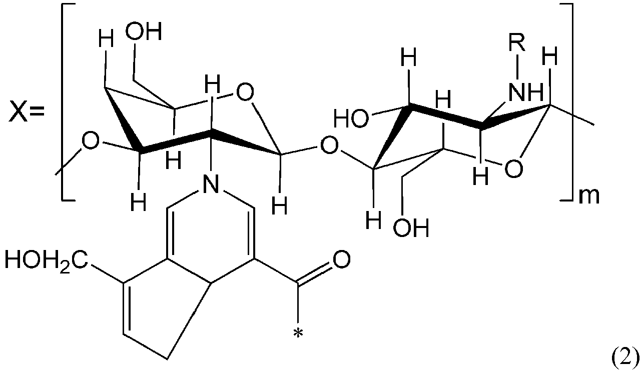 oder aus Formel 3, 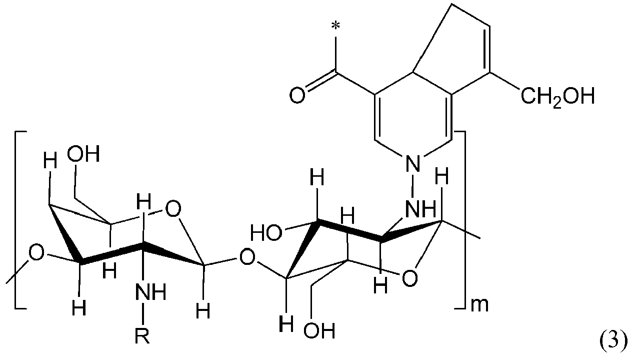 und wobei m und n natürliche Zahlen sind, als eine stationäre Phase in den Heparin-Entfernungsgeräten zur Entfernung von Heparin aus Blut sowie aus den Körperflüssigkeiten der Säugetiere.

2. Die Einsatzweise gemäß Anspruch 1, wobei das Chitosan-Polymer, bei welchem R die Gruppe -CH₂CH(OH)CH₂N⁺(CH₃)₃ ist, in der Reaktion von mit Genipin quervernetztem Chitosan mit Glycidyltrimethylammoniumchlorid gewonnen wird.

3. Die Einsatzweise gemäß Anspruch 1, wobei das quervernetzte Chitosan-Polymer in der Form von Mikrosphären verwendet wird.

4. Die Einsatzweise nach Anspruch 3, wobei die in der Reaktion gewonnenen Hydrogel-Mikrosphären in einer Umkehremulsion eingesetzt werden.

5. Die Einsatzweise nach Anspruch 1, wobei das Chitosan-Polymer in der Form einer dünnen Schicht verwendet wird.

## Revendications

1. Utilisation d'un polymère de chitosane réticulé avec de la génipine de formule générale 1 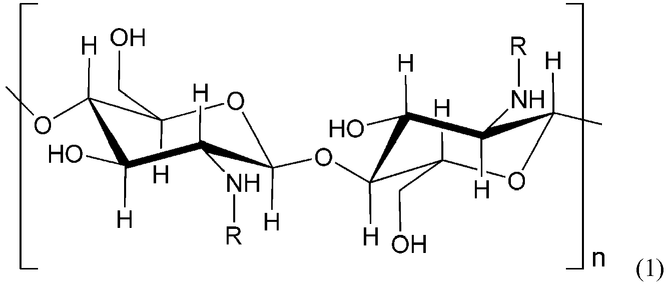 dans lesquels une partie des substituants R désigne -CH₂CH(OH)CH₂N(CH₃)₃ et l'autre partie des substituants R désigne un groupe X de formule 2 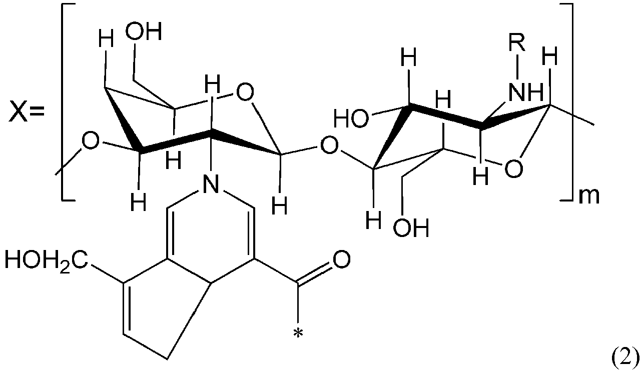 ou de formule 3, 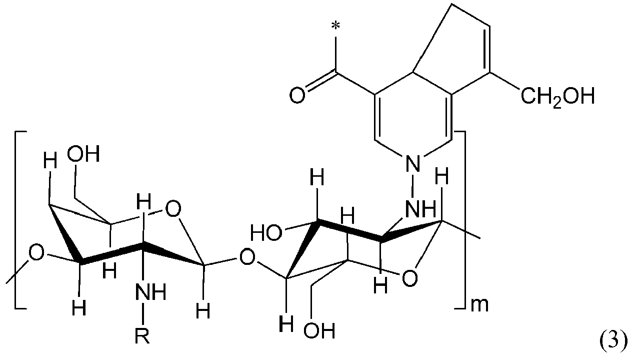 et n et m sont des nombres naturels, en tant que phase stationnaire dans les dispositifs d'élimination de l'héparine pour éliminer l'héparine du sang et des liquides physiologiques des mammifères.

2. Utilisation selon la revendication 1, dans laquelle le polymère de chitosane, où R représente CH₂CH(OH)CH₂N(CH₃)₃ est obtenu dans la réaction du chitosane réticulé avec de la génipine avec du chlorure de glycidyltriméthylammonium.

3. Utilisation selon la revendication 1, dans laquelle le polymère de chitosane réticulé est utilisé sous forme de microsphères.

4. Utilisation selon la revendication 3, dans laquelle on utilise les microsphères d'hydrogel obtenues dans la réaction en émulsion inverse.

5. Utilisation selon la revendication 1, dans laquelle le polymère de chitosane est utilisé sous la forme d'un film.
